Europäisches Patentamt

European Patent Office

Office européen des brevets

(11) Veröffentlichungsnummer : **0 119 394
B1**

(12) EUROPÄISCHE PATENTSCHRIFT

(45) Veröffentlichungstag der Patentschrift :
30.09.87

(21) Anmeldenummer : 84100431.0

(22) Anmeldetag : 17.01.84

(51) Int. Cl.⁴ : **A 61 F   2/38**

(54) Femurteil für eine Kniegelenkprothese.

(30) Priorität : 23.03.83 CH 1595/83

(43) Veröffentlichungstag der Anmeldung :
26.09.84 Patentblatt 84/39

(45) Bekanntmachung des Hinweises auf die Patenterteilung : 30.09.87 Patentblatt 87/40

(84) Benannte Vertragsstaaten :
AT DE FR GB IT NL SE

(56) Entgegenhaltungen :
DE-A- 2 346 973
FR-A- 2 145 957

(73) Patentinhaber : GEBRÜDER SULZER AKTIENGESELL-
SCHAFT
Zürcherstrasse 9
CH-8401 Winterthur (CH)

(72) Erfinder : Horber, Willi     .
Turbinenstrasse 12
CH-8005 Zürich (CH)

(74) Vertreter : Dipl.-Ing. H. Marsch Dipl.-Ing. K. Sparing
Dipl.-Phys.Dr. W.H. Röhl Patentanwälte
Rethelstrasse 123
D-4000 Düsseldorf (DE)

## Beschreibung

Die Erfindung betrifft einen Femurteil für eine Kniegelenkprothese mit zwei Kondylenschalen, die frontal durch einen Patella-Schild miteinander verbunden sind, der seinerseits über ein femorales Plateau in eine die Kondylenschalen dorsal verbindende Rückwand übergeht, die eine konvexe dem Knochen zugewandte Mantelfläche aufweist, wobei die Rückseite des Patella-Schildes ebenfalls eine gegen den Knochen konvexe Mantelfläche bildet.

Femurteile der vorstehend genannten Art sind für sogenannte Teilprothesen bestimmt ; darunter versteht man beim Kniegelenk solche, meist schalenartige Prothesen, für deren Verwendung mindestens funktionsfähige Seitenbänder erforderlich sind. Eine solche Prothese ist beispielsweise aus der DE-A-22 30 734 bekannt ; ihre kappenartige Schale, die aus den natürlichen Kondylen nachgeformten Kondylenschalen und einem diese frontal verbindenden Patella-Schild besteht, wird mit Hilfe eines in den Femur-Markkanal eingesetzten Schaftes in einem Knochenzementbett verankert.

Derartige Prothesen sind jedoch häufig ein ihrem Femurteil nicht mit einem eigentlichen, in den Markhohlraum des Femur reichenden Verankerungsschaft versehen, sondern haben allenfalls auf dem femoralen Plateau der Kondylenschalen Zapfen, die in die Knochen-« Wulste » der Kondylen — gegebenenfalls mit Hilfe von Knochenzement — eingesetzt werden ; so zeigt die DE-A-23 46 973 eine Schalenprothese, zwischen deren Kondylenschalen eine « sattelartige Erhebung » vorhanden ist, die in einen operativ zwischen den Kondylen geschaffenen Schlitz eingreift und ebenfalls mit Hilfe von Knochenzement gehalten wird.

Aufgabe der vorliegenden Erfindung ist es, für Femurteile der genannten Art eine Verankerung zu schaffen, mit der der Femurteil bei zementfreier, allenfalls zementarmer, Verankerung — d. h. einer solchen bei der Knochenzement lediglich als Füllmaterial, aber nicht zur Fixierung dient — mindestens solange primär gehalten wird, bis an- und einwachsendes Gewebe ihn am Knochen fixiert. Diese Aufgabe wird erfindungsgemäss dadurch gelöst, dass die Rückwand mindestens annähernd mit dem Patella-Schild gleichhoch ist, dass ferner das femorale Plateau frei von intramedullär zu verankernden Schäften ist, dass weiterhin die in beiden konvexen Mantelflächen gelegenen, einander am nächsten gegenüberstehenden, geradlinigen Scheitellinien in einer Verbindungsebene liegen, die auf den durch die beiden Scheitellinien gehenden Tangentialebenen senkrecht steht, und. dass schliesslich die Scheitellinien einen nach distal zulaufenden, spitzen Keilwinkel $\alpha$ bilden, für den gilt $0° < \alpha < 30°$.

Die konvexen, gegeneinander gerichteten Mantelflächen von Patella-Schild und Rückwand bilden auf diese Weise einen « Klemmbügel », der während der Operation auf den unter möglichster Schonung der Knochensubstanz entsprechend vorbereiteten Femurknochen aufgeschoben wird. Wie bei bekannten Konstruktionen können dabei auf dem femoralen Plateau zusätzliche, in die Kondylen-Wulste, jedoch nicht in das Knochenmark eindringende Zapfen vorgesehen sein. Die so geschaffene Klemmverbindung gewährleistet eine Primär-Verankerung des Femurteils am Knochen, die im Laufe der Zeit durch ein- und anwachsendes Knochengewebe mehr und mehr verfestigt wird.

Vorteilhaft ist es, wenn die Winkelhalbierende des Keilwinkels $\alpha$ mit der Flächennormalen der durch den Scheitelpunkt der Kondylenflächen gehenden Tangentialebene höchstens einen Winkel $\alpha/2$ bildet ; damit kann gewährleistet werden, dass die Scheitellinien der Mantelflächen höchstens so weit um eine zur Drehachse des Gelenkes parallele Achse gedreht werden, dass eine von ihnen parallel zu einer Ebene verläuft, in der die Femurachse liegt. Es wird dadurch also verhindert, dass die Scheitellinien « überdreht » werden, so dass sie mit der genannten Achsebene einen nach unten offenen spitzen Winkel bilden. Abgesehen von dieser Einengung kann die Lage der Scheitellinien — und damit Form und Lage — beider Konvexflächen weitgehend frei gewählt werden ; insbesondere kann dabei die Verbindungsebene beider Scheitellinien sowohl parallel zur Sagittalebene als auch gegen diese nach medial oder lateral geneigt und/oder um die Flächennormale der durch den Scheitelpunkt der Kondylenschalen gehenden Tangentialebene gedreht sein.

Weiterhin ist es zweckmässig, wenn der Patella-Schild bezüglich der Sagittalebene zwischen den Kondylenschalen unsymmetrisch ausgebildet ist, um der Unsymmetrie natürlicher, linker und rechter Kniegelenke mindestens annähernd Rechnung zu tragen.

Für die Herstellung des neuen Femurteils eignen sich alle in der Implantat-Technik verwendeten Materialien und Materialkombinationen, sofern sie die notwendige elastische Nachgiebigkeit aufweisen ; bevorzugte Werkstoffe sind daher Metalle und Metall-Legierungen, insbesondere Kobalt- oder Titan-Legierungen, faserverstärkte Kunststoffe, sowie Kohlenstoff und Karbidverbindungen.

Im folgenden wird die Erfindung anhand von Ausführungsbeispielen im Zusammenhang mit der Zeichnung näher erläutert.

Figur 1 zeigt schematisch einen Schnitt I-I von Fig. 2, der in der Sagittalebene zwischen den Kondylenschalen verläuft, die neue Konstruktion an einem Femurknochen gehalten ;

Figuren 2 und 3 sind eine Aufsicht und eine Ansicht von Fig. 1 von oben bzw. von links ;

Figur 4 ist ein schematische Darstellung der zur Ansicht von Fig. 1 entgegengesetzten « Grenz »-Stellung der Scheitellinien ;

Figur 5 gibt schematisch einen Femurteil wieder, bei dem die Verbindungsebene der Scheitellinien gegen die Sagittalebene verdreht ist ;

Figur 6 schliesslich ist eine Ansicht in Richtung dorsal/ventral eines Femurteils, bei dem die Verbindungsebene der Scheitellinien gegen die Sagittalebene nach medial/lateral geneigt ist.

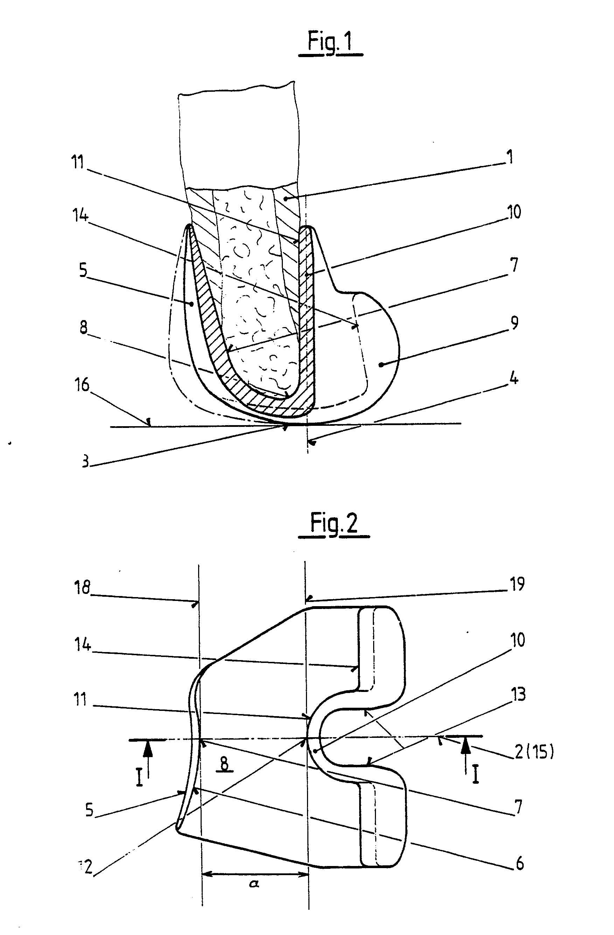

Der am distalen Ende des Femurknochens 1 (Fig. 1) zu fixierende Femurteil hat beidseitig einer Sagittalebene 2 (Fig. 2) je eine an ihrer Aussenseite kondylenartig ausgebildete Schale 3, die bei einer Bewegung des Kniegelenks auf einem nicht gezeigten Tibiateil gleitend abrollt und dabei im wesentlichen die Belastungskräfte überträgt. Die Flächennormale zur in Streckstellung des Gelenkes im wesentlichen horizontalen Tangentialebene 16 im Scheitelpunkt der Kondylenflächen der Schalen 3 ist mit 4 bezeichnet.

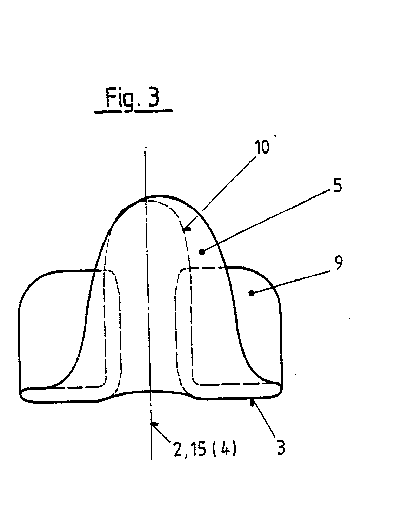

Auf der in Fig. 1 und 2 nach links gerichteten Frontseite des Gelenkes sind die Kondylenschalen 3 über einen Patella-Schild 5 miteinander verbunden, der in diesem Beispiel einen Teilausschnitt aus einer Mantelfläche eines Hohlzylinders darstellt. Bezüglich der Sagittalebene 2 ist der Patella-Schild 5, dessen konvexe Mantelfläche 6 dem Knochen 1 zugewandt ist, derart asymmetrisch ausgebildet, dass der Flächenanteil des Patella-Schildes 5 lateral, d. h. zur Aussenseite hin, grösser ist als nach medial. Die Fig. 2 und 3 zeigen somit einen für einen linken Femurknochen 1 bestimmten Femurteil.

Der Patella-Schild 5 geht über ein femorales Plateau 8 in eine Rückwand 10 über, die die — in Fig. 1 nach rechts gelegenen — dorsalen Bereiche 9 der Kondylenschalen 3 untereinander verbindet. Diese Rückwand 10 besteht in diesem Beispiel ebenfalls aus einem Teilausschnitt eines Hohlzylinders, der in Richtung Flächennormalen 4 aufrecht steht und dessen konvexe Mantelfläche 11 dem Knochen 1 zugewandt ist. Die einander am nächsten gegenüberliegenden Mantellinien der beiden konvexen Mantelflächen 6 und 11 sind die Scheitellinien 7 und 12 ; sie verlaufen erfindungsgemäss so, dass sie in einer Verbindungsebene 15 liegen, sich also nicht in getrennten, räumlich sich kreuzenden Ebenen erstrecken. Ferner bilden sie in diesem Beispiel einen Keilwinkel α von 15° miteinander. Die Verbindungsebene 15 ihrerseits steht senkrecht auf den beiden durch die Scheitellinien 7 und 12 gehenden Tangentialebenen 18 und 19 und fällt in diesem Beispiel mit der Sagittalebene 2 zusammen.

Die etwa halbzylindrische Schale der Rückwand 10 geht auf der dem Knochen 1 abgewandten Aussenseite der Prothese in gerade, vertikale Seitenwände 13 der dorsalen Bereiche 9 der Kondylenschalen 3 über. Der den Patella-Schild 5 erzeugende Hohlzylinder hat in diesem Beispiel einen erheblich grösseren Krümmungsradius als der Hohlzylinder der Rückwand 10 ; die Krümmungsradien sind dabei so gewählt, dass eine Anpassung der konvexen Mantelflächen 6 bzw. 11 an die Form des Femurknochens in den zugehörigen Bereichen erleichtert wird.

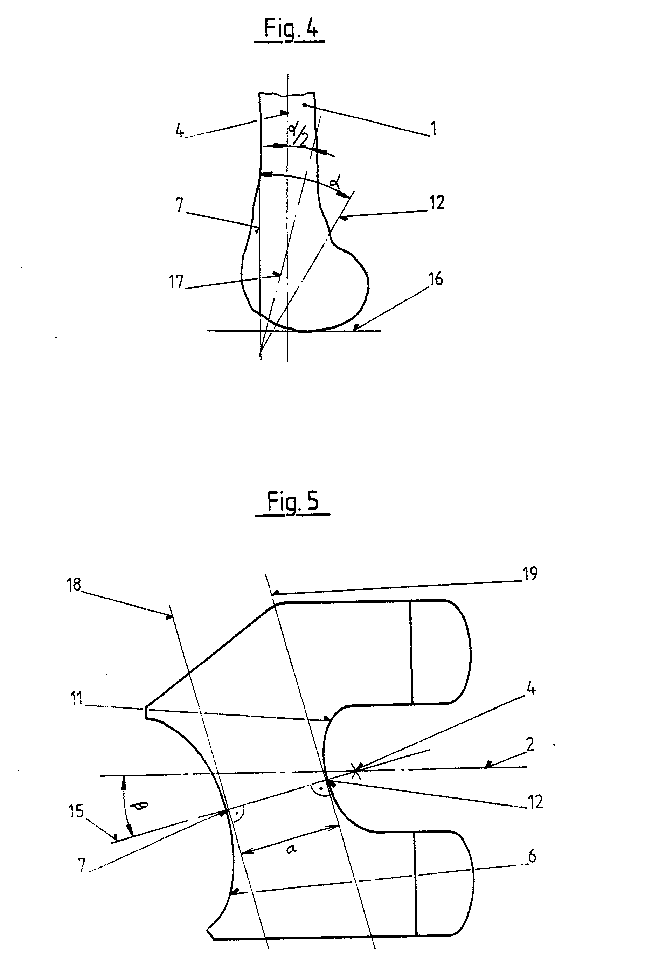

Um den Klemmbügel-Effekt zu gewährleisten, sind die Scheitellinien 7 und 12 Geraden, die, wie bereits erwähnt, einen Winkel einschliessen, der bis zu 30° betragen kann und im vorliegenden Beispiel 15° ist ; in dem gezeigten Beispiel verläuft die Scheitellinie 12 — als ein Grenzfall der erwähnten Forderung nach Anspruch 2 — in Richtung der Flächennormalen 4, während die Scheitellinie 7 dagegen nach frontal geneigt ist ; selbstverständlich ist es auch möglich, dass beide Scheitellinien 7 und 12 bzw. ihre Projektionen auf die Sagittalebene 2 — wenn sie selbst diese Ebene unter einem Winkel schneiden — gegen die Flächennormale 4 geneigt sind, oder dass — im anderen Grenzfall — die Scheitellinie 7 parallel zur Flächennormalen 4 verläuft. Dies ist schematisch in Fig. 4 verdeutlicht, aus der auch die Bedingung zu entnehmen ist, dass dafür die Winkelhalbierende 17 des Keilwinkels α mit der Flächennormalen 4 einen Winkel α/2 bildet.

Die am Knochen 1 anliegenden Innenflächen 14 der dorsalen Bereiche 9 der Kondylenschalen 3 sind, wie in Fig. 1 angedeutet, gegen die Flächennormale 4 ebenfalls leicht geneigt ; diese Neigung hat den Zweck, zum einen ein unbeabsichtigtes « Abrutschen » des Femurteils vom Knochen 1 zu erschweren und zum zweiten zu ermöglichen, dass die künstlichen Kondylenschalen 3 bei minimaler Knochenresektion oben relativ weit wieder nach vorn gezogen werden können. So wird ein grosser Abrollbereich für ein extremes Beugen des Kniegelenks geschaffen.

Wie bereits erwähnt und besonders deutlich aus Fig. 3 zu entnehmen ist, ist der Patella-Schild bezüglich der Sagittalebene 2 unsymmetrisch ausgebildet, obwohl seine Scheitellinie 7 (Fig. 2) in dieser Ebene verläuft. Der grössere Flächenanteil des Patella-Schilds ist dabei lateral der Ebene 2 gelegen. Die Asymmetrie hat den Vorteil, eine gegenüber einem symmetrischen Schild verbesserte Annäherung an die physiologischen Gegebenheiten für die auf dem Schild « gleitende » Patella zu erreichen.

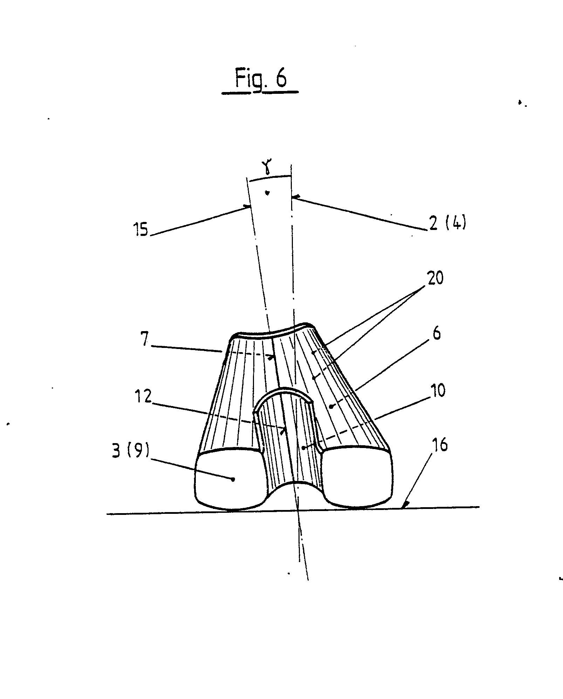

Bezüglich der Form der Mantelflächen 6 und 11 sei noch erwähnt, dass diese Form nicht auf Körper mit kreiszylindrischen Querschnitten beschränkt ist, sondern auch elliptische, ovale oder andere, z. B. auch polygonale, Querschnittsformen möglich sind und dass die Radien dieser Querschnittsformen sich nach distal hin vergrössern können, so dass die Mantellinien 20 wie in in Fig. 6 kegelförmig divergieren ; die Scheitellinien 7 und 12 mit kürzestem Abstand a (Fig. 2 und 5) definieren jedoch immer eine Verbindungsebene 15, die im ersten Ausführungsbeispiel mit der Sagittalebene 2 zusammenfällt. Die Verbindungsebene 15 und damit die den geschilderten Klemmeffekt vor allem bewirkenden Scheitellinien 7 und 12 können jedoch schräg zur Sagittalebene 2, d. h. nach lateral oder medial geneigt, verlaufen und/oder gegen diese Ebene 2 um die Flächennormale 4 als Drehachse verdreht sein ; der Drehwinkel β kann dabei von der Sagittalebene 2 aus nach jeder Seite 0° bis 20° betragen. Fig. 5 stellt rein schematisch eine solche Drehung dar, während in Fig. 6 eine gegen die Sagittalebene 2 geneigte Verbindungsebene 15 der Scheitellinien 7 und 12 wiedergegeben ist. Der Neigungswinkel γ kann dabei nach medial Werte bis 5°, nach lateral bis 12°, annehmen. Selbstverständlich sind auch Ausführungsformen des neuen Femurteils möglich, bei denen die Verbindungsebene 15 gleichzeitig geneigt und verdreht ist.

**Patentansprüche**

1. Femurteil für eine Kniegelenkprothese mit zwei Kondylenschalen (3), die frontal durch einen Patella-Schild (5) miteinander verbunden sind, der seinerseits über ein femorales Plateau (8) in eine die Kondylenschalen (3) dorsal verbindende Rückwand (10) übergeht, die eine konvexe dem Knochen (1) zugewandte Mantelfläche (11) aufweist, wobei die Rückseite des Patella-Schildes (5) ebenfalls eine gegen den Knochen (1) konvexe Mantelfläche (6) bildet, dadurch gekennzeichnet, dass die Rückwand (10) mindestens annähernd mit dem Patella-Schild (5) gleichhoch ist, dass ferner das femorale Plateau (8) frei von intramedullär zu verankernden Schäften ist, dass weiterhin die in beiden konvexen Mantelflächen (6, 11) gelegenen, einander am nächsten gegenüberstehenden, geradlinigen Scheitellinien (7, 12) in einer Verbindungsebene (15) liegen, die auf den durch die beiden Scheitellinien (7, 12) gehenden Tangentialebenen (18, 19) senkrecht steht, und dass schliesslich die Scheitellinien (7, 12) einen nach distal zulaufenden, spitzen Keilwinkel α bilden, für den gilt 0° < α < 30°.

2. Femurteil nach Anspruch 1, dadurch gekennzeichnet, dass die Winkelhalbierende des Keilwinkels α mit der Flächennormalen (4) der durch den Scheitelpunkt der Kondylenflächen (3) gehenden Tangentialebene (16) höchstens einen Winkel α/2 bildet.

3. Femurteil nach Anspruch 1 oder 2, dadurch gekennzeichnet, dass die Verbindungsebene (15) der Scheitellinien (7, 12) gegen die Sagittalebene (2) nach medial oder lateral geneigt ist.

4. Femurteil nach einem der Ansprüche 1-3, dadurch gekennzeichnet, dass die Verbindungsebene (15) der Scheitellinien (7, 12) gegen die Sagittalebene (2) um die Flächennormale (4) der durch den Scheitelpunkt der Kondylenflächen (3) gehenden Tangentialebene (16) gedreht ist.

5. Femurteil nach einem der Ansprüche 1-4, dadurch gekennzeichnet, dass der Patella-Schild (5) bezüglich der Sagittalebene (2) zwischen den Kondylenschalen (3) unsymmetrisch ausgebildet ist.

**Claims**

1. A femoral part for a knee joint replacement having two condyle shells (3) frontally interconnected by a patella plate (5) merging by way of a femoral plateau (8) into a rear wall interconnecting the condyle shells (3) dorsally, the rear wall having a convex generated surface (11) near the bone (1), the back of the patella plate (5) also forming a convex generated surface (6) near the bone (1), characterised in that the rear wall (10) is at least substantially of the same height as the patella plate (5), the femoral plateau (8) is free from stems requiring intramedullary anchorage, the straight crest lines (7, 12) which are disposed in the two convex generated surfaces (6, 11) and which are closely opposite one another are disposed in a connecting plane (15) perpendicular to the tangential planes (18, 19) passing through the two crest lines (7, 12), and the crest lines (7, 12) form an acute wedge angle (α) which narrows in the distal direction and for which 0° < α < 30°.

2. A femoral part according to claim 1, characterised in that the angle bisector of the angle α forms an angle of at most α/2 with the perpendicular (4) to the tangential plane (16) passing through the crest of the condyle surfaces (3).

3. A femoral part according to claim 1 or 2, characterised in that the connecting plane (15) of the crest lines (7, 12) is inclined in the median or lateral direction towards the sagittal plane (2).

4. A femoral part according to claims 1-3, characterised in that the connecting plane (15) of the crest lines (7, 12) is rotated relatively to the sagittal plane (2) around the perpendicular (4) to the tangential plane (16).

5. A femoral part according to any of claims 1-4, characterised in that the patella plate (5) is devised asymmetrically relatively to the sagittal plane (2) between the condyle shells (3).

**Revendications**

1. Partie fémorale d'une prothèse d'articulation rotulienne, présentant deux calottes condyliennes (3) reliées frontalement l'une à l'autre par l'intermédiaire d'un écusson de rotule (5) se prolongeant à son tour, par l'intermédiaire d'un plateau fémoral (8), par une paroi postérieure (10) qui relie dorsalement les calottes condyliennes (3) et qui présente une surface périphérique convexe (11) tournée vers l'os (1), la face postérieure de l'écusson de rotule (5) formant également une surface périphérique (6) convexe par rapport à l'os (1), caractérisée par le fait que la paroi postérieure (10) se trouve au moins approximativement à la même hauteur que l'écusson de rotule (5) ; par le fait que le plateau fémoral (8) est par ailleurs dépourvu de tiges destinées à un ancrage intramédullaire ; par le fait que les lignes droites (7, 12) des sommets, situées en vis-à-vis le plus proche et s'étendant dans les deux surfaces périphériques convexes (6, 11), se trouvent en outre dans un plan de liaison (15) s'étendant perpendiculairement aux plans tangentiels (18, 19) passant par les deux lignes (7, 12) des sommets ; et par le fait que les lignes (7, 12) des sommets forment enfin un angle aigu de conicité α s'étendant vers la région distale, et obéissant à la relation 0° < α < 30°.

2. Partie fémorale selon la revendication 1, caractérisée par le fait que la bissectrice de l'angle de conicité α forme au maximum un angle α/2 avec la normale superficielle (4) du plan tangentiel (16) passant par le sommet des surfaces condyliennes (3).

3. Partie fémorale selon la revendication 1 ou 2, caractérisée par le fait que le plan de liaison (15) des lignes (7, 12) des sommets est incliné, par rapport au plan sagittal (2), vers la région médiale ou latérale.

4. Partie fémorale selon l'une des revendications 1-3, caractérisée par le fait que le plan de liaison (15) des lignes (7, 12) des sommets est décalé, par rapport au plan sagittal (2), autour de la normale superficielle (4) du plan tangentiel (16) passant par le sommet des surfaces condyliennes (3).

5. Partie fémorale selon l'une des revendications 1-4, caractérisée par le fait que l'écusson de rotule (5) est réalisé asymétriquement par rapport au plan sagittal (2) entre les calottes condyliennes (3).

## Fig.1

## Fig.2

## Fig. 3

2,15 (4)

## Fig. 4

## Fig. 5

## Fig. 6